# EUROPEAN PATENT SPECIFICATION

(11) **EP 1 016 031 B1**
(45) Date of publication and mention of the grant of the patent: **22.08.2007**
(21) Application number: 98911470.7
(22) Date of filing: 02.03.1998
(51) Int. Cl.: G06K 9/00, G06T 1/00

(54) **METHOD AND APPARATUS FOR ACQUIRING AND RECONSTRUCTING MAGNIFIED SPECIMEN IMAGES FROM A COMPUTER-CONTROLLED MICROSCOPE**
VERFAHREN UND GERÄT ZUM ERFASSEN UND WIEDERHERSTELLEN VON VERGRÖSSERTEN PROBEBILDERN DURCH EIN RECHNERGESTEUERTES MIKROSKOP
PROCEDE ET APPAREIL POUR ACQUERIR ET RECONSTRUIRE DES IMAGES ECHANTILLONS AGRANDIES A PARTIR D'UN MICROSCOPE COMMANDE PAR ORDINATEUR

(30) Priority: 03.03.1997 US 805856; 27.02.1998 US 32514
(43) Date of publication of application: 05.07.2000
(73) Proprietor: BACUS LABORATORIES, INC., Lombard, IL 60148 (US)
(72) Inventor: BACUS, James, V., Downers Grove, IL 60515 (US); BACUS, James, W., Oakbrook, IL 60521 (US)
(74) Representative: HOFFMANN EITLE
(86) International application number: PCT/US1998/004208
(87) International publication number: WO 1998/044446

(56) References cited:
- EP-A- 0 209 422
- EP-A- 0 246 010
- US-A- 4 175 860
- US-A- 4 287 272
- US-A- 5 252 487
- US-A- 5 257 182
- US-A- 5 428 690
- US-A- 5 544 650
- SZELISKI R: 'IMAGE MOSAICING FOR TELE-REALITY APPLICATIONS' PROCEEDINGS OF THE IEEE WORKSHOP ON APPLICATIONS OF COMPUTER VISION, XX, XX 01 May 1994, pages 44 - 53, XP000861632
- KRISHNAN A ET AL: "Panoramic image acquisition" PROCEEDINGS 1996 IEEE COMPUTER SOCIETY CONFERENCE ON COMPUTER VISION AND PATTERN RECOGNITION (CAT. NO.96CB35909), PROCEEDINGS OF IEEE CONFERENCE ON COMPUTER VISION AND PATTERN RECOGNITION, SAN FRANCISCO, CA, USA, 18-20 JUNE 1996, pages 379-384, XP002178054 1996, Los Alamitos, CA, USA, IEEE Comput. Soc. Press, USA ISBN: 0-8186-7258-7

## Description

### Field of the Invention

This invention relates to a method of and apparatus for acquiring and recording digital images of an optical image viewed through a computer-controlled automated microscope and also, to using the latter in a quantitative analysis of plant or biological specimens.

### Background of the Invention

In the image analysis and quantification of DNA from tissue sections as disclosed in United States Patent 4,741,031, and also especially in the immunohisto-chemistry assays on the kinds of cell analysis systems disclosed in United States Patents 5,086,476; 5,202,931; and 5,252,487 issued to Bacus, there is a problem of first locating the cancer regions for analysis under low power and then remembering them when performing the analysis under higher power. There is a need, a requirement to image and digitally record an object in a relatively flat plane at high resolution/magnification. Today, it is impractical to construct an optical image sensor large enough to cover the entire image area e.g., of a specimen on a microscope slide, at the required resolution. This is because lens size and resolution/magnification issues limit the size of the field of view of magnified objects and their resulting images. Viewing through a microscope is akin to viewing through a periscope in that one sees a very small field of view even at low magnifications, such as 1.25X. A pathologist using a microscope often scans a slide to obtain in his mind an overall view or sense of what constitutes the specimen and he remembers the general locations of the diagnostically significant, small pieces of the specimen. Usually, these are the diseased areas, such as malignant or potentially malignant portions of the specimen. To obtain higher resolution and magnification of these suspicious portions, the pathologist switches to a higher magnification objective lens but then the field of view becomes much smaller again. Often, the pathologist switches back and forth between the lower magnification, larger field of view objective lens to orient himself relative to the specimen and the high magnification, smaller field of view to obtain the detailed, high resolution view of the suspicious area on the specimen. Thus, the user never receives a magnified, condensed overall view of the specimen or a portion of the specimen but must remember the series of views taken at low magnification. Likewise, at high resolutions and high magnifications, the user never receives or views a collection of adjacent images but must interrelate these successive images in the user's mind.

Stated differently, the problem is that once the microscope is set up for quantitation by image analysis under, e.g. 40x, where all of the diaphragms are set and light adjusted, etc., if the operator needs to move to another tissue area, it is first desirable to locate it at e.g. 10x. In fact, often regions can only be located at this power. In order to do so however, all of the settings (diaphragms, light levels, wavelengths of light, etc.) have to be changed to view the tissue at this magnification. Currently, there is no way to ensure that one could go back to the settings at the previous 40x magnification and continue on with the quantitative image analysis of that same specimen. This necessitates finding those areas under 40x, without changing objectives, which is very slow and time-consuming, and often important cancer areas can be missed.

Also, another problem with tissue analysis, at its current state-of-the-art, is that it is not completely automated, for example, with regard to finding structural regions such as glands, basal layers or other important diagnostic regions. However, as set forth in US6031930 , if these regions are located, important very sensitive diagnostic measurements can be performed. For example, as disclosed in the aforesaid patent application, assays are made of a variety of tissue types, both human and animal for analysis of neoplasia in tissue, for pre-invasive cancer in tissue, and the effects on the tissue of chemopreventive agents. A quantitative analysis by image processing techniques is performed on tissue types, having various architectural features, such as breast tissue, colon tissue, prostate tissue, esophageal tissue, skin tissue, cervix tissue, etc. These tissues have different morphologies, and they undergo different neoplasias usually resulting from a cellular mutation, as may be enhanced by a carcinogen, or resulting from a cellular proliferation rate enhanced by hormones, growth factors, or other inducers of abnormal tissue growth. Often it is desired to quantify small changes in the neoplasia when it is incipient or through a series of analyses performed at close time intervals to measure whether the neoplasia progression is increasing or has been slowed, stopped or regressed.

Usually, the tissue specimens are cut to expose the basal layer for review under the microscope. Typically, the quantitative measurements are performed at 40x to obtain 100 to 400 tissue images. The 40x objective provides a narrow field of view of a very small portion of the entire basal layer. Often, the basal layer is somewhat elongated and generally linear such as a basal layer in a rat esophagus; and the analysis of the basal layer requires examining it along its length. The basal layer in a mouse colon is more in the form of an irregular, circular shape; and the analysis of this basal layer requires traveling about this circular shape. In breast tissue samples, suspected tumor areas may be at widely-spaced points in the stained tissue; and one wants to be able to navigate and travel to these specific suspected areas and to do the 40x analysis at these areas in an efficient manner. There is a need to allow an experienced operator to interact with the analysis to locate and identify such regions in an interactive manner. Especially, an interactive manner that would be familiar and consistent with the use of a microscope manually, with high power magnification and low power magnification simultaneously available, but performed on a computer terminal. Such a level of interaction is different than the interaction with the system disclosed in the above-listed Bacus Patents. There is a need to take the level of interaction to a higher level and let each component, the human and the computer, perform the part that it does best, in the most cost-effective manner.

The current state of archiving the digital images achieved through a microscope is often by having photographs or by video tapes. The photographs are difficult to use as is a video tape particularly when the user wants to move rapidly back and forth between various images and to scroll through various adjacent parts of the specimen image. Further, current archival methods lack an overall macro image of the specimen, which allows the user to know exactly where the particular high resolution view is from when the user is making an analysis of the high resolution image.

While digitized images can be stored magnetically or otherwise digitized and recorded on various recording mediums, no current archival system allows the user to toggle back and forth between high magnification images and low magnification images or between various images at different magnifications such as that achieved by a pathologist switching microscope objective lenses in real time to get the macro and micro images from the same location on the specimen. Heretofore, the practice of pathology has been relatively limited to the use of microscopes and to the pathologist having to use the microscope to review the particular specimen.

There is a need for a dynamic system whereby one or more or several pathologists, including a consulting pathologist, may view the same area simultaneously and interact with one another either in diagnosis or in analysis. Also, it would be best if the images from the specimen could be stored so that a pathologist could easily examine the images at his leisure using an intranet or Internet browser at a later date merely by accessing the particular web site where the images are located.

A similar problem exists on the Internet or intranet where a pathologist may receive a single field of view magnified image taken from a specimen over the Internet or the intranet on his browser. The pathologist must be provided with explanations to coordinate the high resolution view with the lower resolution view. The number of views available to the pathologist is very limited, and the pathologist is unable to select other views or to scroll to neighboring views at the areas that are most interesting to the pathologist.

There are available on the market computer-controlled, automated microscopes such as those sold by Carl Zeiss, Inc., Thornwood, N.J., under the name Axioplan 2 for taking photographic images of a specimen in the microscopic field of view. Those particular microscopes have computer-controlled and automatically adjusted subsystems, such as an illumination subsystem, a focusing subsystem, a diaphragm or optical stops subsystem, an objective lens subsystem, or a filtering subsystem. As an operator selects changes from one objective lens, such as one providing low magnification, e.g., 4x, to a higher magnification, e.g., 40x, the computer-automated system will turn the lens turret to switch in the high magnification automatically and adjusts the lens and also automatically adjusts the illumination to eliminate glare and to provide the proper light illumination including light density. Further, the focus is adjusted, and the proper diaphragm openings are automatically reset. Thus, the computer-controlled, automated subsystems automatically reset to values stored and predetermined for each selected objective lens and the analysis being done.

Those particular microscopes can be used to view various objects or specimens, but are most typically used to view and to take still photographs of biological specimens, such as tissues and cells. Those particular microscopes lack a computer-controlled X and Y stage for translating a specimen-carrying slide with respect to the field of view of the selected objective lens. Currently, pathologists and others who use such microscopes want to view the specimen images in full color or in enhanced colors using fluorescent illumination and/or monochromatic images using the automated filter subsystems on the microscopes. Currently trained pathologists or clinicians are accustomed to manually adjust and have a microscope available to them to view larger areas of the specimen at low magnification, and then to momentarily switch in a new higher magnification lens to obtain a more highly magnified image of a portion of the specimen viewed at low magnification. Pathologists and those working in this area have created in themselves a desire to view suspect tissue as though they were viewing the specimen tissue through a microscope and appear to resist analysis systems that do not provide them this ability.

The microscopic field of view reduces very substantially as the magnification increases. The skill level of the clinician and/or pathologist is important to locate viewing the most suspicious areas or points of interest on the specimen. Sometimes, a technician will do a first assay and analysis. A pathologist will return to the selected points of interest or other points of interest for review and analysis. One concern with respect to a quantitative analysis of breast cancer tissue or prostate biopsy tissue samples to pap smears or other tests for various cancers or the like is that a particularly suspicious point in the tissue may be overlooked and missed during the visual assay or for selection for an automated review analysis. When observing at high magnifications, the field of view is limited to very small area of the specimen. Hence, the observer has difficulty in knowing and remembering the actual, exact location of this small periscopic view within the very large whole specimen.

Often, also the problem is finding or locating the tissue or cells for view at high magnification so that artifacts and/or blank spaces on the slide are not viewed. A number of approaches have been proposed to prescreen and locate by an X and Y address the cells or small points of interest from a very large number of potential points of interest.

There are currently available commercial services for prescreening pap smears where one can mail in slides and the service will do a microscopic prescan at high magnification for suspected or suspicious areas of interest which are marked and given address locations, and also a video tape of the slide specimen is returned by this service to the sender. The sender then reviews the areas of interest located during the prescreening and/or the video tape to complete the analysis.

In an attempt to locate and allow review of specified points of interest, U.S. Patent No. 5,428,690 to Bacus discloses a system for prescreening of a field of cells on a specimen slide at low magnification before the viewer. When seeing a point of interest to be viewed at high magnification, the viewer will operate a switch or the like to select and record the address of these selected prescreened points of interest. Later, these prescreened points of interest are then brought into position to be analyzed at high magnification. This system is fairly or too slow for many uses.

A very expensive system is currently in use in which a pathologist located at a diagnostic center is able to make a diagnostic opinion with respect to specimens under a microscope at a remote center. The pathologist at the diagnostic center manipulates robotic controls to send telepathy signals via a special, dedicated satellite or other large bandwidth channel to control the microscope at the remote site in approximately real time. The pathologist then can maneuver the remote microscope to shift the microscope's field of view and to send, by telepathy, a highly magnified, very small image back to the pathologist. This system requires each subscriber to have a special microscope operable by manipulation of the robotic controls at the diagnostic center and a dedicated or large bandwidth channel to convey real time video signals and hence results in a very high cost for the assay being done. To assist the pathologist in staying within the specimen at the remote site, a peripheral edge or map of the specimen is made using a second video camera and a light box or by using computerized scanning equipment to trace the outline or peripheral edge of the specimen boundaries. A small circle of light is displayed within the map of the specimen so that the pathologist at the diagnostic center knows the location of the field of view of the highly magnified image within the specimen. In a sense, the pathologist is operating in real time in the same way that he would use his own microscope at his diagnostic center except for a slight transmission delay used to transmit the video signals of the highly magnified image over large bandwidth channel. Although the pathologist has a small map or peripheral outline of the specimen, the pathologist's field of view of the actual specimen is only the small circle of view that is coming through the microscope objective lens. This does not help the pathologist locate suspicious areas of interest as in a prescreening of the entire tissue. The pathologist may switch to the lowest magnification to get the largest field of view of a small section of the specimen, but he never views the entire specimen at any magnification. Also, there is no image analysis quantitative testing from the received images at the diagnostic center; and no quantitative assaying is done with these images at the diagnostic center.

There is a particular interest today in using the Internet system because it is so readily accessible by users at a low cost and using a computer and viewing screen connected to the computer. One problem with trying to do any transmission of digitized, microscopic, highly magnified images over the Internet is that the bandwidth is too narrow to accommodate the tremendous amount of stored data which needs to be transmitted. There is a need for a system which would allow a pathologist or another person, to be able to perform tissue analysis or quantitative assays using a standard computer terminal from a location remote from the automated microscope system that digitized the optical images.

In the article "Image acquisition of microscopic slides" by Earl Henderson and James R. Seamans which appeared in "Proceedings of the SPIE" Vol. 2173 pages 21-27 published by the International Society for Optical Engineering, there is described a system in which a sample on a microscope slide is imaged at several different magnifications, including a low magnification "base image" and higher magnifications. At the higher magnifications the images are each only of a small segment of the sample. These image segments, which overlap by 50%, are knitted together by an auto-correlation algorithm_to form a high definition image file of the sample at each of the higher magnifications.

In the articles "Implementing a Collaboratory for Microscopic Digital Anatomy", by Young, S. J. et al. which appeared in the International Journal of Supercomputer Applications and High Performance Computing, Summer-Fall 1996 USA Vol. 10 pp. 170-181 and "Telemicroscopy", by Fan G. Y. that appeared in Ultramicroscopy December 1993 pp. 499-503, there is described research work intended to make a High Voltage Electron Microscope situated at the University of California in San Diego available by remote control and viewing to other researchers at other locations via the Internet. The remote control includes being able to set microscope magnification. The images are transmitted back as a mosaic to be reassembled and displayed at the remote location.

In accordance with an embodiment, a person such as a pathologist at a computer terminal may view on a computer screen or monitor digitized images of a microscope specimen at different magnifications as selected by the person. Further, the person may receive on the screen a low magnification, reconstructed image of the entire specimen to aid the person in interactively selecting points of interest on the specimen, such as along a basal layer of a tissue specimen.

The MAC 2000 Configuration Manual, Revision 1-2 April 1995, Ludt Eletronic Products Ltd, describes the MAC 2000 as a modular system for providing automation control. The system can be configured with different modules to suit different tasks, for example with microscopes. Several systems are defined as products using the MAC 2000 control unit. Motorized XY(Z) stages are the most common configuration for the MAC 2000. The MAC 2000 can be configured also as a photometer controller, filter/shutter controller, and an autofocus controller - to name a few. Any combination of the previous is also possible. The manual explains the possible configuration options for each type of module in the MAC 2000.

### Summary of the Invention

According to the present invention, there is provided a method as set out in claim 1.

The present invention also provides a system as set out in claim 12.

Optional features are set out in the other claims.

More specifically, and in accordance with an embodiment, the microscope's small field of view limitation of a specimen is overcome by providing to the viewer a reconstructed, digitized and magnified image of the entire specimen (or a very large portion of the specimen) for performing a visual analysis of the entire tissue in full color to aid in the selection of points of interest to be viewed at a higher magnification and higher resolution. This is achieved by acquiring and storing a large number of low magnification images of the specimen through a microscopic scanning system, e.g., 35 image tiles of the specimen at 1.25X, and then assembling and coordinating these stored tiled images to form an overall, low magnified image of the specimen, i.e., a macro image of the specimen. Preferably, the digitized, stored macro image is reduced in size by a software system to a smaller size, e.g., a 1/4 size image and it is this reduced in size macro tiled image that is displayed on a local screen or is sent over a low band width or a high band width channel to a remote screen. The pathologist not only does not need to have others do a slow laborious prescreening to locate suspicious areas for analysis or for viewing at high magnification, he can use his own experiences to enable him to go directly to the most suspicious areas which he sees on the macro image. He can, on a priority basis, do the most suspicious area first, followed by lower priority areas of interest .

In accordance with an embodiment, there is provided a new and improved microscopic display system that displays the low magnification composite image of the specimen to allow the user to view and to interactively select points of interest, each of which may be displayed at high magnification and resolution. This is achieved by providing the user with a marker, such as a cursor or the like, to select the defined area of interest from the stored, macro image; and to acquire reproduced, spatially adjacent high resolution, digitized images of the selected area of interest. More specifically, the specimen, when it was first scanned at low magnification to provide a macro view of the specimen, the addresses or locations of the tile images and/or pixels for the composite image were acquired. Therefore, any selected region of interest in the macro image has locations to which the microscopic stage may be automatically repositioned under a changed, higher magnification lens to acquire higher magnification, digitized image tiles that can be assembled into a micro image. Herein, both the macro and micro images are formed of adjacent digitized image tiles which have been coordinated to reproduce spatially the original image that was scanned.

It is the high magnification images, usually at 40x, that were analyzed using image processing techniques as disclosed in the aforesaid patent application, to provide an assay or numerical histological data for the specimen.

In accordance with the preferred embodiment of the invention, the pathologist may desire to see a larger region for analysis at high resolution than can be accommodated at this magnification on his high magnification viewing screen. The pathologist can quickly change the view on his screen to view adjacent, highly magnified, digitized image tiles at this high resolution by scrolling up or down or right to left to shift these digitized, adjacent image tiles into view on the screen. Thus, even at a higher magnification of a region the pathologist is able to obtain a much larger view than the small field for the objective lens in use of adjacent tissue or cells to give him a broader, overall perspective of what is happening or what has happened in specific section of a specimen. For instance, a pathologist may want to see at high magnification and to assay at this high magnification, a suspicious area, the pathologist can draw a mark about the area and cause it to then be assayed and displayed.

By having displayed on a low magnification screen or split screen of the full composite area, by having the high magnification region being marked on the low image screen, and by having the region being scrolled to view adjacent high magnification images on the high magnification screen, the pathologist has available information to guide him in the sense of helping to navigate intelligently within the specimen in his search for cancerous tissue or the like for further inspection or measurement of malignant characteristics. Often, when restricted to a field of view of an objective microscope, the pathologist has a difficult time, in the words of a cliche, of seeing the forest for the trees. In the present invention, the pathologist has a full, magnified, reduced in size specimen view with the higher image area marked thereon to guide him and to let him see the forest. He can also see a region of the forest at higher magnification by scrolling adjacent tree images onto the high magnification screen.

In accordance with a further embodiment, the user may elect to view an intermediate magnification and resolution. In the preferred system, at least three magnification images are acquired and stored as digitized images. The intermediate resolution images may be acquired by either switching automatically to a new objective lens and acquiring new digitized image tiles at the intermediate magnification or by using software to reconstruct a new intermediate digitized image from the existing high and low magnification, digitized images.

The preferred, low magnification image, which is reduced in size, can be transmitted over narrow band width channels such as a local area network or over the Internet through various servers and computers. Likewise, the reconstructed, high magnification images can be transmitted over such narrow band width channels.

The preferred microscope is fully computer controlled, a pathologist or other person having a split screen computer such as a PC, can be connected to the microscope and operate it from a remote location with the computer microscope system to digitize and construct the macro image. Likewise, the pathologist can navigate to points of interest and with the computer microscope system to digitize and construct the desired micro images. With an embodiment, there is no need for a specialized microscope at each remote location nor for a broad band channel to send video signals in real time between the diagnostic center and the remote location.

### Brief Description of the Drawings

FIG. 1 is a screen view of a system embodying the present invention showing a low magnification image of a specimen on a microscope slide in one window, a high magnification image of a portion of the low magnification image selected by a region marker and a control window;
FIG. 2 is a view of a display screen of the apparatus embodying the present invention showing the control window a low magnification window having a plurality of high magnification micro image regions delineated therein and a high magnification window including one or more of the micro image regions;
FIG. 3 is a view similar to FIG. 2 including the control window but also including a low magnification region from the slide showing regions marked by a histology grade or structure through automatic analysis of tissue and a high magnification window showing markings related to the grading or histology grade yielded by the automatic analysis of tissue in combination with a window showing a numerical score;
FIG. 4 is a block diagram of the apparatus embodying the present invention;
FIG. 5 is a block diagram of a portion of the apparatus shown in FIG. 4 showing details of a mechanical arrangement of a microscope;
FIG. 6 is a flow diagram related to operation of the apparatus;
FIG. 7 is a flow diagram of details of one of the steps in FIG. 6;
FIG. 8 is a display screen showing control parameters to be manipulated thereon;
FIG. 9 is a flow chart for a region outlying routine;
FIG. 10 is a flow chart for a scanning and analyzing routine;
FIG. 11 is a schematic showing of the limits of travel of the microscope stage with respect to the image tiles;
FIG. 11A is a perspective view of the microscope stage and stepper motors and encoders providing a closed loop drive for the motors;

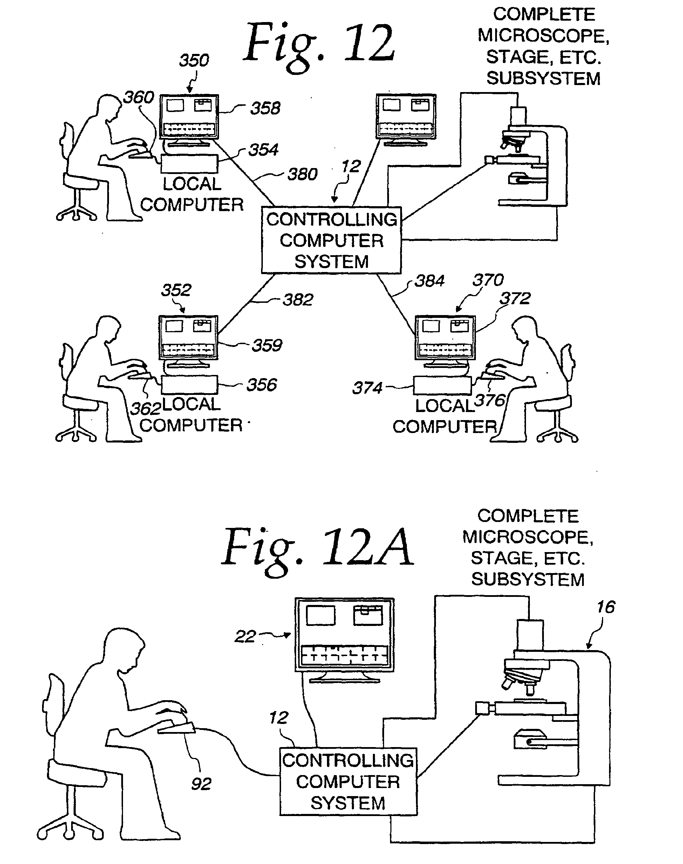

FIG. 12 is a block diagram of a networked system allowing multiple workstations to obtain access to diagnostic image information and to manipulate such information locally at each workstation;
FIG. 12A is a view of the system described in connection with FIG. 5;

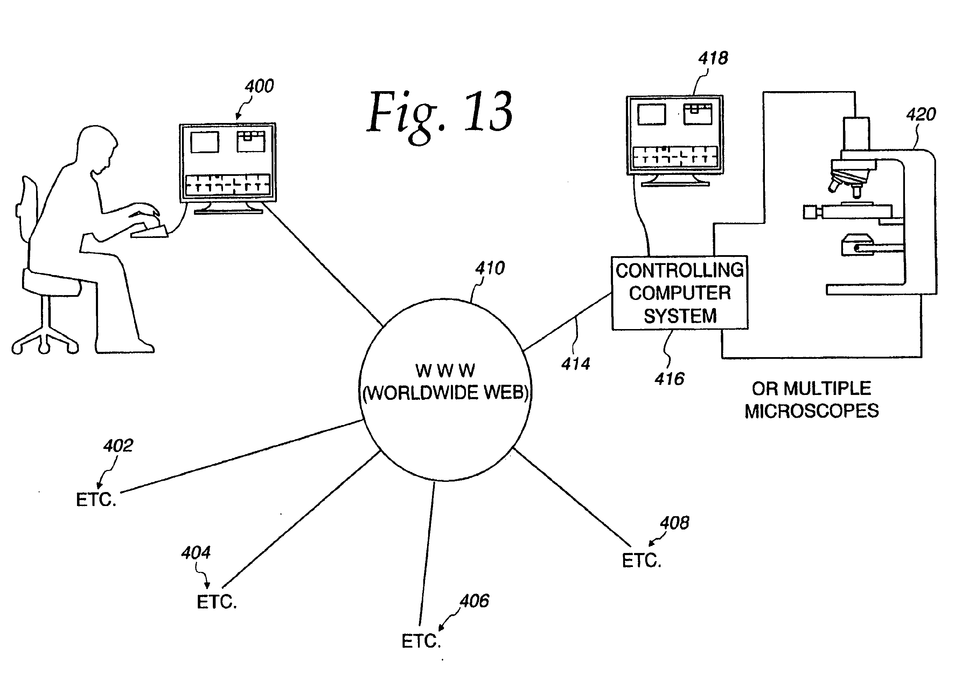

FIG. 13 is a block diagram of a remote networked system for distributing and accessing diagnostic images and data through a hypertext transport protocol based server directly or over a packet network;
FIG. 14 is a view of a low magnification, reconstructed image from a basal layer of rat esophagus;
FIG. 14A is a view of a high magnification, reconstructed image from a selected point of interest from FIG. 14;
FIG. 15 is a view of a low magnification image of a mouse colon having a basal layer;
FIG. 15A is a view of a reconstructed macro image of a mouse colon;
FIG. 16 is a schematic view of an analysis from regions of a basal layer;
FIG. 16A is a schematic view of an analysis to provide to a Z score; and
FIG. 17 is a schematic view showing texture analysis tests for regions.

### Detailed Description of the Preferred Embodiment

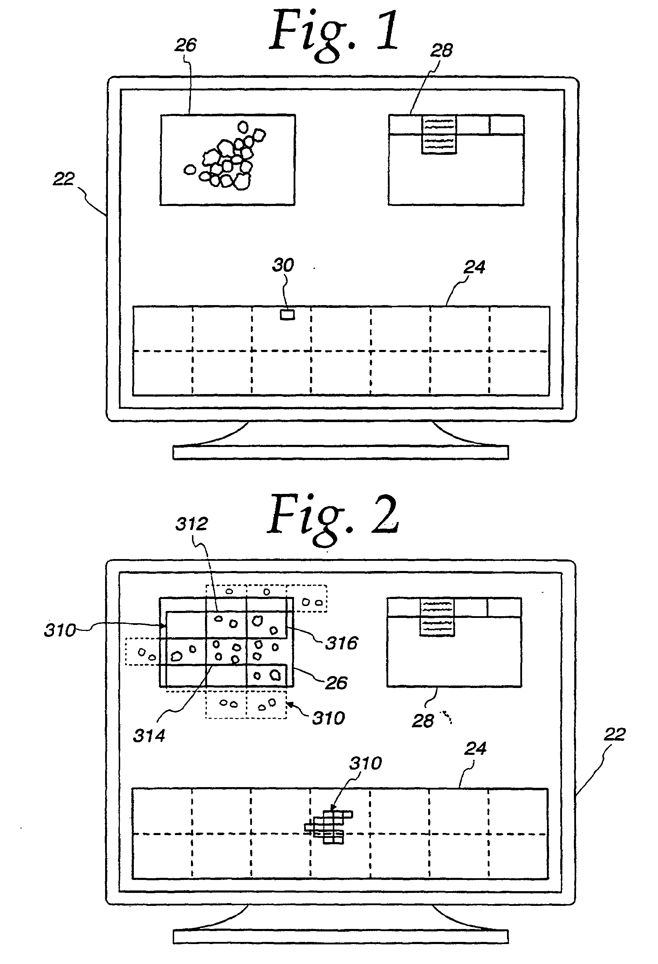

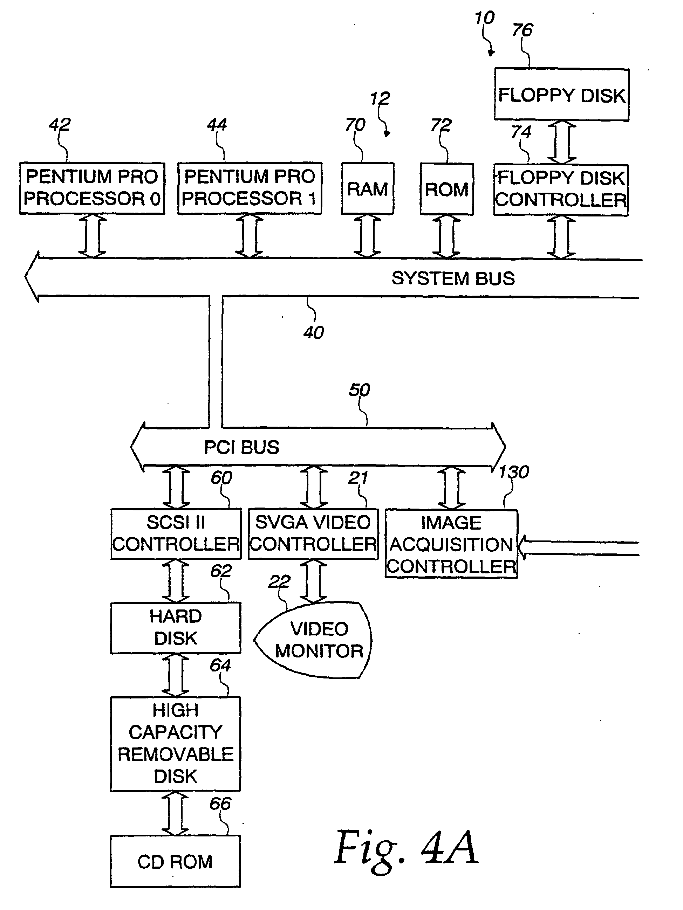

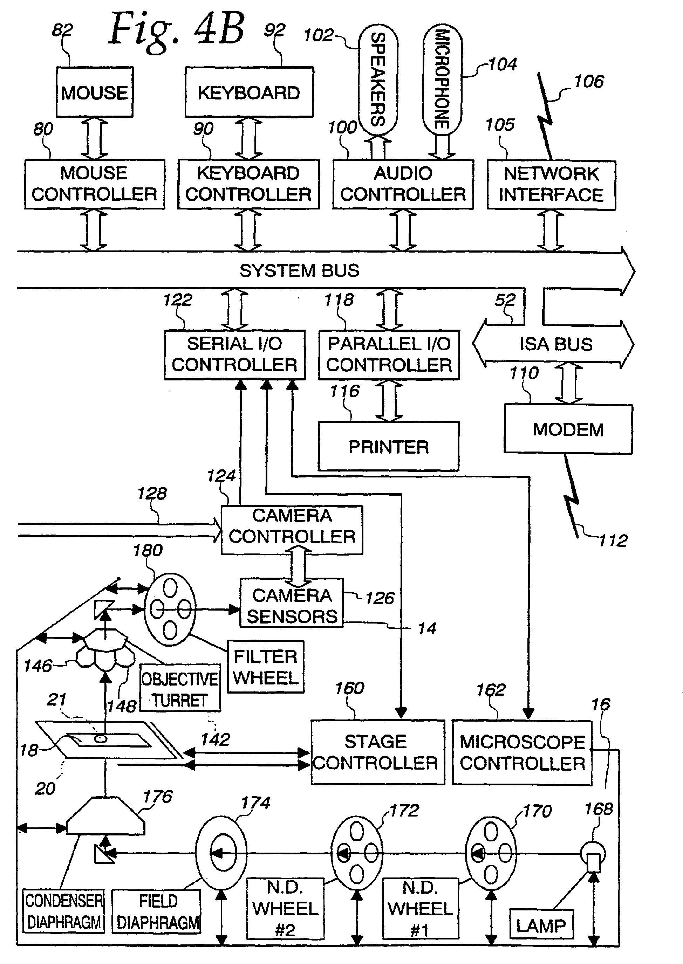

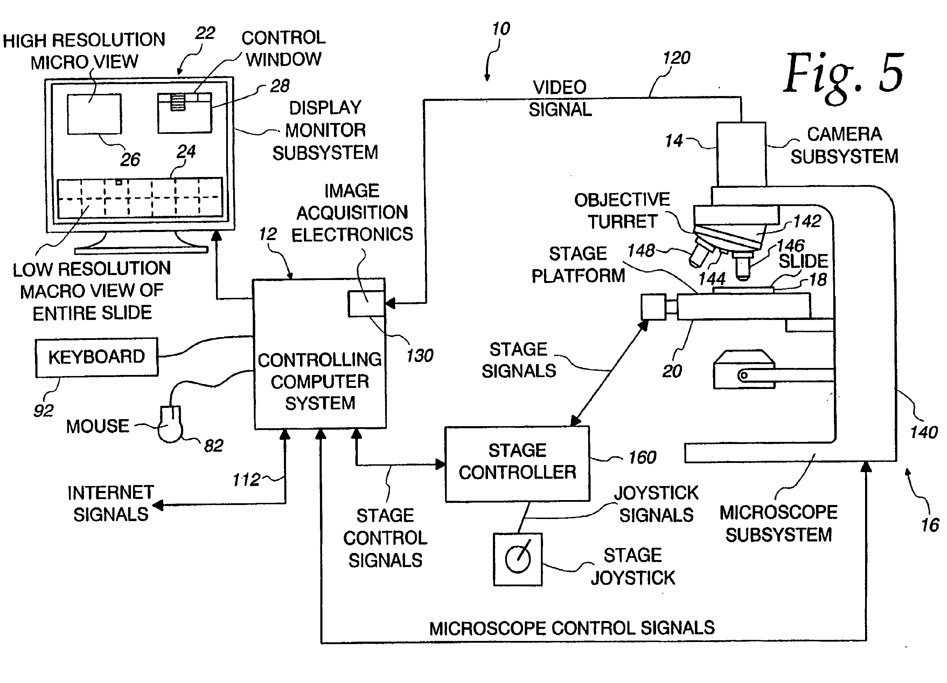

Referring now to the drawings, and especially to Figs. 4 and 5, apparatus for synthesizing low magnification and high magnification microscopic images is shown therein and generally identified by reference numeral 10. The system includes a computer 12 which is a dual Pentium Pro personal computer in combination with a Hitachi HV-C20 video camera 14 associated with a Zeiss Axioplan 2 microscope 16. The computer system 12 is able to receive signals from the camera 14 which captures light from the microscope 16 having a microscope slide 18 positioned on an LUDL encoded motorized stage 20. The encoded motorized stage 20 includes a MAC 2000 stage controller for controlling the stage in response to the computer 12. A microscope slide 18 includes a biological specimen 21 which is to be viewed by the microscope and whose image is to be digitized both at low magnification and at high magnification as selected by a user. The low magnification digitized image is then displayed on a 21 inch Iiyama video display monitor 22 having resolution of 1600 by 1200 to provide display screens of the type shown in Figs. 1 through 3 including a low magnification image 24, for instance, at 1.25 power, a high magnification image 26, for instance at 40 power and a control window or image 28. The low magnification image may have identified therein a region 30 which is reproduced at high magnification in high magnification screen or window 26 so that a pathologist or other operator of the system can review architectural regions of interest in low magnification image 24 and simultaneously view them in high magnification in the high magnification screen or window 26 to determine whether the cells forming a portion of the architectural feature need be examined further for cancer or the like or not.

The computer 10 is constructed around a PCI system bus 40 and has a first Pentium Pro microprocessor 42 and a second pentium pro microprocessor 44 connected thereto. The system bus 40 has connected to it a PCI bus 50 and an ISA bus 52. The PCI bus 50 has a SCSI controller 60 connected thereto to send and receive information from a hard disk 62. The hard disk 62 also is coupled in daisy chain SCSI fashion to a high capacity removal disk and to a CD Rom drive 66. The hard disks 62 contains the programs for operating the system for controlling the microscope 16 and for processing the images as well as for doing a quantitative analysis of the selected portions of the histological specimens being viewed on the slide 18. The system bus 40 also has connected to it a random access memory 70 within which portions of the program being executed are stored as well as a read only memory 72 for holding a bootstrap loader as well as portions of the basic input/output operating system. A floppy disk controller 74 is coupled to the system bus 40 and has connected to it a floppy disk drive 76 for reading and writing information to a floppy disk as appropriate. A mouse controller 80 is coupled to the system bus and has a mouse 82 which operates as a pointing device for controlling manipulations on the screen 22 and within the windows 24, 26 and 28. A keyboard controller 90 is connected to the system bus and has a keyboard 92 connected thereto. The keyboard 92 may be used to send and receive alpha numeric signals to other portions of the computer. An audio controller 100 has a plurality of speakers 102 and a microphone 104 connected thereto for audio input and output and is coupled to the system bus 40. A network interface, such as a network interface card 104, is connected to the system bus and can provide signals via a channel 106 to other portions of a network or internet to which the system may be connected. Likewise, signals can be sent out of the system through a modem 110 connected to the ISA bus 52 and may be sent via a channel 112, for instance, to the internet. A printer 116 is connected via a parallel I/O controller 118 to the system bus in order to provide printouts as appropriate of screens and other information as it is generated. A serial I/O controller 122 is connected to the system bus and has connected to it a camera controller 124 which is coupled to CCD sensors 126 in the cameras. The CCD sensors 126 supply pixel or image signals representative of what is found on the slide 18 to an Epix pixci image acquisition controller 130 coupled to the PCI bus 50.

The microscope 16 includes a base 140 having a stage 20 positioned thereon as well as an objective turret 142 having a plurality of objectives 144, 146 and 148 thereon. The objective 144, for instance, may be of 1.25x objective. The objective 146 may be a 20X objective. The objective 148 may be a 40X objective. Signals from the camera sensors and controller are supplied over a bus 128 to the image acquisition system where they are digitized and supplied to the PCI bus for storage in RAM or for backing storage on the hard disk 62.

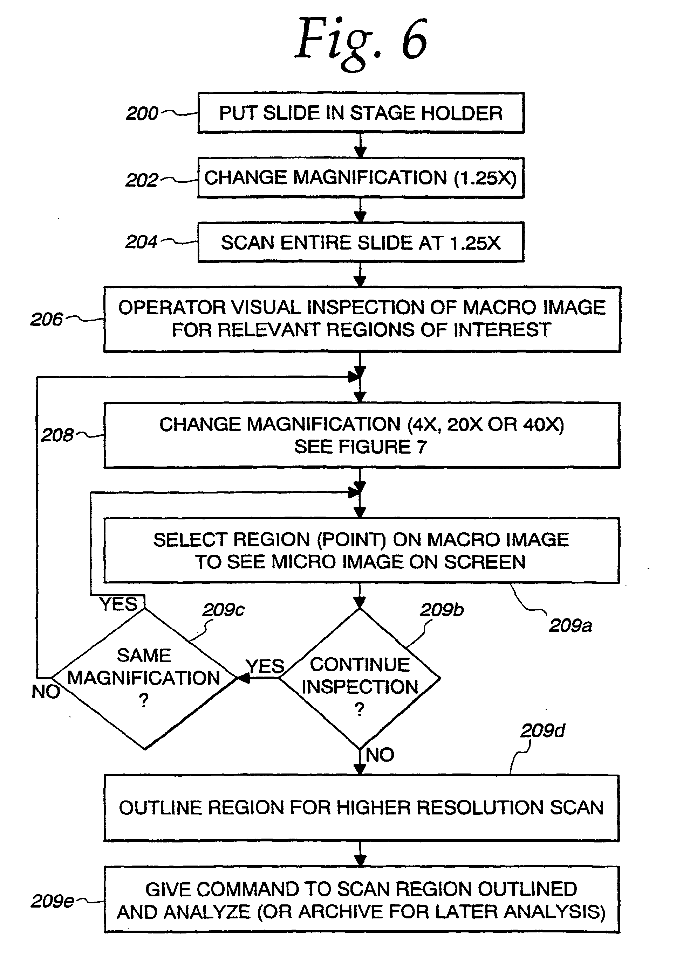

When a specimen is on the slide 18 the stage 20 may be manipulated under the control of the computer through a stage controller 160 coupled to the serial I/O controller 122. Likewise, a microscope controller 162 controls aspects of the microscope such as the illumination, the color temperature or spectral output of a lamp 168 and the like. For instance, in normal operation, when a specimen is placed on the slide, specimen slide 18 is placed on the stage 20 in a step 200, as shown in FIG. 6, the processors 42 or 44 send a command through the system bus to cause the serial I/O controller 122 to signal the microscope controller to change magnification to 1.25X in a step 202. This is done by rotating the objective turret of the Axioplan 2 microscope to select the objective 144. Likewise, the controller sets the color temperature of the lamp 168, sets a pair of neutral density filter wheels 170 and 172 and sets a field diaphragm 174 for the correct illumination. A condenser diaphragm 176 is also controlled and a color filter wheel 180 may also be controlled to apply the appropriate filter color to the CCD censors 126 in the camera. The entire slide is then scanned in a step 204. The images are tiled and melded together into the overall image 24 supplied on the screen 22 to provide the operator in the step 206 with a visually inspectable macro image of relevant regions of the slide of interest.

In order to provide the magnified image, the mouse may be moved to identify a marker segment or region which, for instance, may be a rectangular region (as shown as 30 in FIG. 1) which will cause the microscope to change magnification as at step 208 to 4x, 20x, 40x, etc., by rotating the turret to bring the appropriate objective lens system into viewing position.

Next the user, in a step 209a, uses the mouse to select the region on the macro image in order to select the micro image to be viewed on the screen 22. In a step 209b a test is made to determine whether the user has commanded continued inspection. If the user has, a test is made in a step 209c to determine if the magnification is to be changed by changing the selected objective. In the event the magnification is to be changed control is transferred to the step 208. If the magnification is to remain unchanged control is transferred to the step 209a. In the event inspection is not to continue the region selected is outlined for higher magnification scan in a step 209d. In a step 209e, a command may be received to scan or acquire the higher magnification image for display in screen 26. The image may then be archived for later analysis, displayed or analyzed immediately.

In order to perform the magnification called for in step 208, the overall illumination and control of the microscope will be controlled so that in a step 210 the objective turret 142 will be rotated to place the higher power objective above the slide 18. In a step 212 voltage to the lamp will be changed to adjust the lamp 168 to provide the proper illumination and color temperature as predetermined for the selected objective. In a step 214, the condenser diaphragm 176 will have its opening selected as appropriate to provide the proper illumination for that objective. In a step 216, the filter turret 180 will select the proper light wavelength filter to be supplied to the camera sensors. For instance, a red, blue or green filter, as appropriate, particularly if the specimen has been stained. In a step 218 the field diaphragm 174 will have its opening changed. In a step 220 the neutral density filter wheel 170 will select a neutral density filter and in a step 222 the neutral density filter wheel 172 will also select a neutral density filter. In a step 224 the X, Y and Z offsets will be used for reconstruction of the recorded image at the magnification and in a step 226 the current position will be read from encoders in the stage which are accurate to .10 micron.

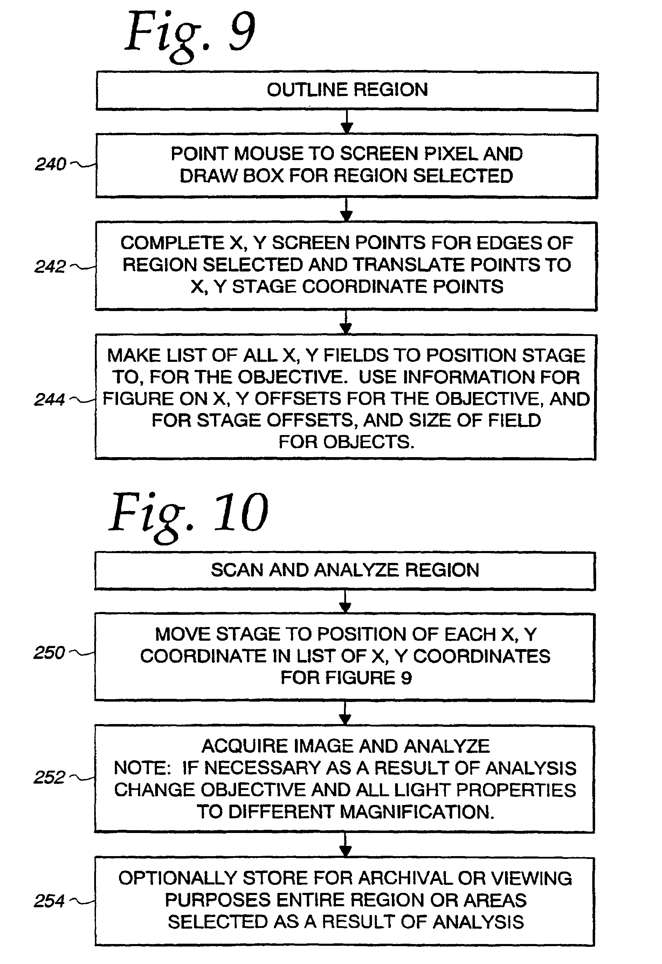

In order to identify the selected region the mouse is moved to that area of the region in a pointing operation in a step 240 as shown in FIG. 9. The mouse may be moved to draw a box around the region selected. In a step 242 the X and Y screen points are computed for the edges of the regions selected and the computed image or pixel points are translated to stage coordinate points in order to control the stage of the microscope. In a step 244 a list of all of the X fields for positioning the stage for the objective is stored in random access memory and may be backed up on the hard disk. The information from the X offsets for the objective and the stage offsets is used as well as the size of the field to position the slide properly under the objective to capture the micro image.

When the slide has been positioned properly, as shown in FIG. 10 in a step 250 the stage is positioned for each of the X and Y coordinate values in stage coordinate values and the digitized image is captured by the cameras and stored in RAM and backed up on the hard disk. The image may be then analyzed quantitatively in various manners such as those set forth in the previously-identified United States application. Optionally the image may be stored for archival purposes in a step 254.

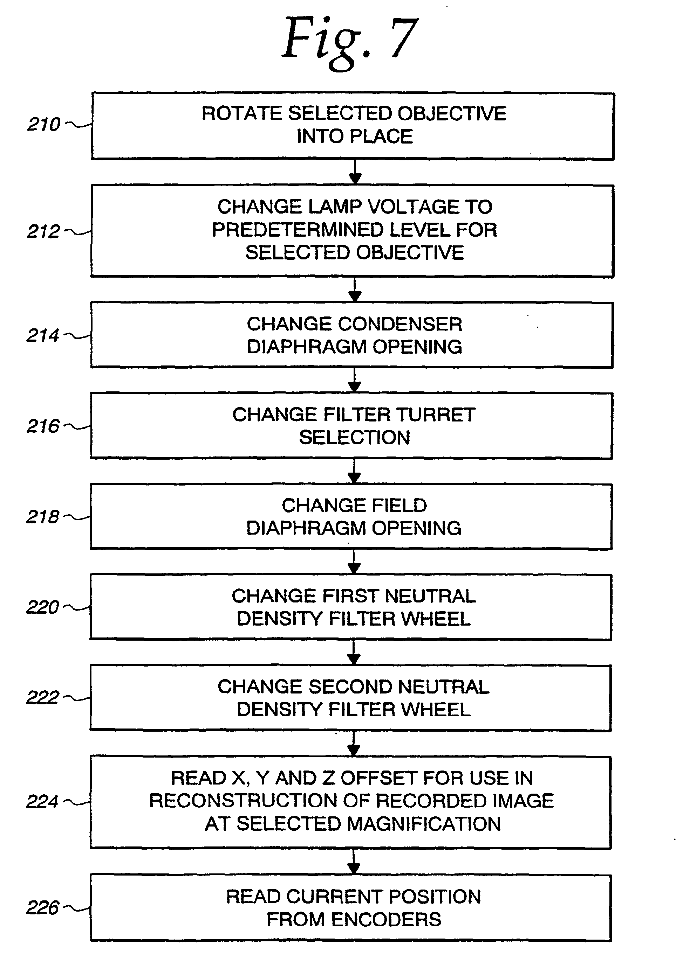

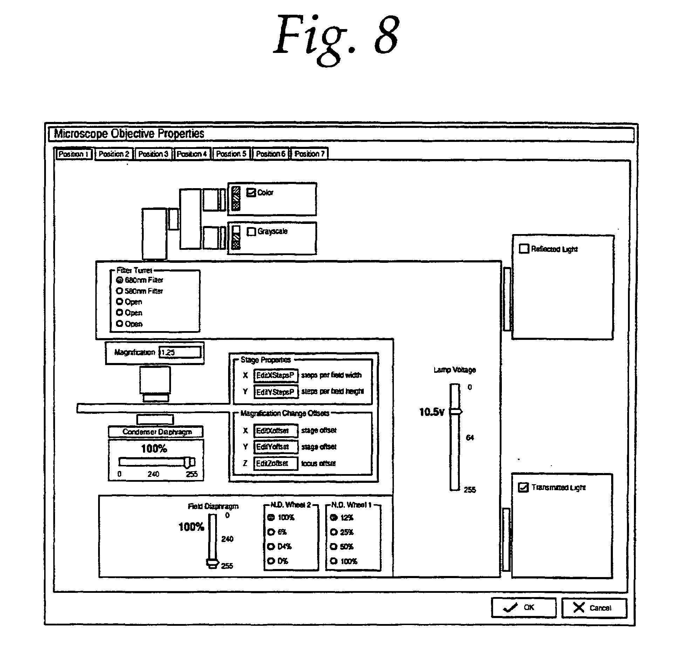

In order to override the specific control functions that take place as shown in FIG. 7, a screen is provided as shown in FIG. 8 wherein the XY step size can be edited, the X, Y and Z offset can be edited, the lamp voltage can be selected, the neutral density filter can be selected as well as the opening of the field diaphragm and several other microscopic characteristics. FIG. 8 is a view of the settings of the microscope objective properties of the Axioplan 2, computer-controlled microscope.

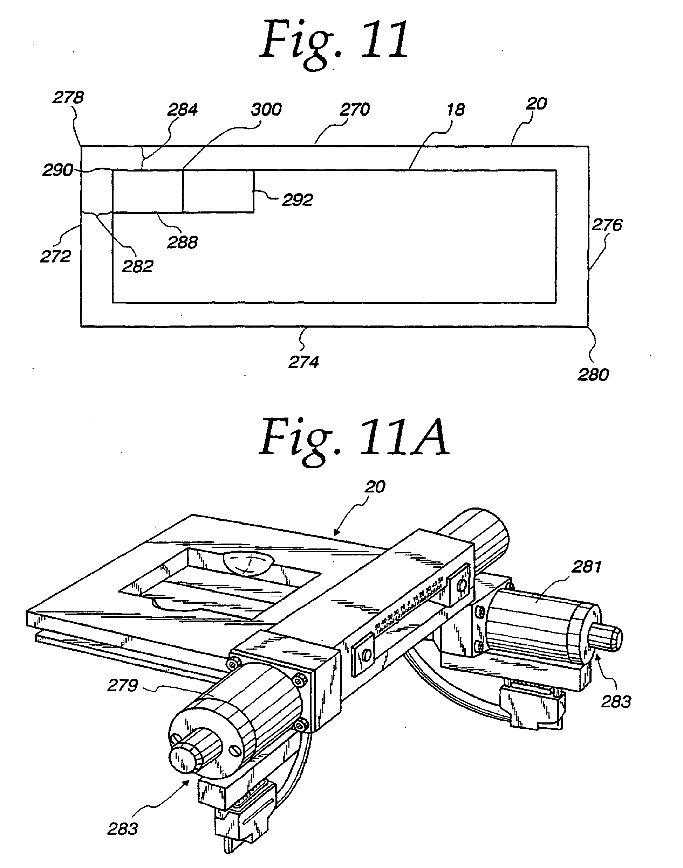

The X and Y positioning is specifically carried out as shown in FIG. 11 where the slide 18 is shown with a slide boundary 270, 272, 274 and 276. Stage boundary for limits of the stage travel for purposes of the stage the stage can be moved all the way from an upper left hand corner of travel 276 to a lower right hand corner of travel 280. At the upper left hand bounded corner of travel 278 limits which a signal that the end of travel has been reached and the stage is then translated a short distance 282 in the extra action and a short distance 284 in the Y direction to define the first tile 288 in terms of a reference point 290 at its upper left hand corner. Since the size of the macro image tile 288 is known, the next macro image tile 292 may be placed contiguous with it by moving the stage appropriately and by measuring the location of the stage from the stage in counters without the necessity of performing any image manipulation. The image tiles 288 and 292 may be abutted without any substantial overlap or they may be overlapped slightly, such as a one pixel with overlap, which is negligible insofar as blurring of any adjacent edges of abutted image tiles. The upper left hand corner 300 of the tile 292 defines the rest of 292 and other tiles can be so defined. Micro image tiles can likewise be defined so that they are contiguous but not substantially overlapping, as would interfere with the composite image. This avoids the problems encountered with having to perform extended computations on digital images in a frame storer or multiple frame storage in order to match or bring the images into contiguity without blurriness at the edges of contiguous image tiles. It may be appreciated as shown in FIG. 2 that the low power image 24 has a plurality of micro images defined therein which are tiled and which are shown in higher magnification as individual tiles 312, 314, 316 and the like in FIG. 2. In addition, the region 310 when magnified as shown in the window 26 may exceed the bounds of the window and thus the window may include scroll bars or other means for allowing the image 310 which is larger than the window 26 to be examined from within the window 26.

The stage 200 is best seen in FIG. 11A and includes the X and Y stepper motors 279 and 281 with their respective encoders, which provide a closed loop system to give the .1 micron accuracy versus the usual 5 or 6 micron accuracy of most microscope stages without a closed loop system. This closed loop system and this very high accuracy allow the abutting of the tile images for both high magnification and low magnification images without the substantial overlap and the time-consuming and expensive software currently used to eliminate the overlap and blurriness at the overlapping edges of adjacent image tiles. With the precisely positioned stage and by using the tiling system described in connection with FIG. 11, where the slide is precisely positioned relative to a center point CP for the slide, and the known position of point 278 is always taken from the same point, the tiles may be positioned precisely in a horizontal row and precisely in vertical rows to reconstruct the macro image and the micro image. This reconstruction is done without the use, as in the prior art, of extensive software manipulation to eliminate overlapping image tiles, horizontally or vertically or the haphazard orientation of image tiles.

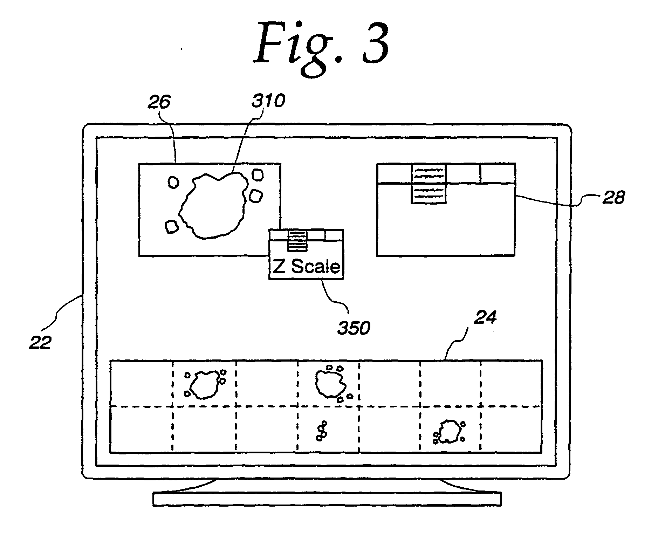

Furthermore, as shown in FIG. 3, the low power window 24, high power window 26 and control window 28 can be used in conjunction with reporting of quantitative analysis data, histograms, etc. for the specimen being viewed; and such analysis information may be provided as a visual output in a window 320. Each of the various regions 30 that a pathologist may follow in marking various features in the low power window 24 and the high power window 26 may be reflected in both windows in order that an audit trail is provided for the system.

The present invention also includes the facility for allowing remote diagnostics to occur by being able to couple the system either over a network communication facility to an intranet, for instance via the network interface, or via a modem or other suitable connection, to an internet so that once the image has been scanned and stored in memory on hard disks or other storage, remote users may be able to access the low magnification image as well as the high magnification image and move around within both images to make determinations as to the histological characteristics of the samples via Z scores.

An additional feature of the system includes a plurality of networked workstations coupled to a first computer console 12 having a display screen 22 connected to the microscope 14. Satellite work stations 350 and 352 are substantially identical to the work station 12 including respective computers 354 and 356 coupled to displays 358 and 360. The devices can be manipulated through input devices 360 and 362 which may include a keyboard, mouse and the like. Also a third device can be connected including a work station 370, having a display 372, a computer 374 and an input device 376. Each of the devices is connected over respective network lines 380, 382, 384 to the computer 12 which transmission may be via either net or the like. Each of the different operators at the physically separate viewing stations can locate regions from the view of entire tissue cross sections via a macro view and label the regions for subsequent scanning and/or quantitative analysis. A single operator at the instrument station 12 can locate regions to view the entire tissue cross section. Those regions can be labeled for subsequent scanning and/or quantitative analysis with subsequent review and physically remote viewing stations, for instance, in an operating room or in individual pathologists' signout areas in order to review analysis results while still maintaining and reviewing the entire macro view of the tissue and/or the individual stored images from which the quantitative results were obtained. The viewing stations 350, 352 and 370 can comprise desk top computers, laptops, etc. There is no need for a microscope at the network stations 350, 352 and 370.

In a still further alternative embodiment, remote workstations 400, 402, 404, 406 and 408 may be connected through a server 410 which may be supplied via a packet switched network. The server 410 and may be a hypertext transport protocol based server of the type used for the World Wide Web or may be a telnet type server as used previously in internet remote operation applications. The server 410 communicates via a communications channel 414 with a local computer 416 having a display 418 associated therewith, the local computer 416 being connected to the microscope 420. Each of the remote work stations 400, 402, 404, 406 and 408 may perform the same operations as the stations 350, 352 and 370 although they do it from nearby buildings or even from around the world, thus providing additional flexibility for others to make use of the specimen obtained and being viewed under the microscope 420. In addition, stored images may be disseminated through the server 410 to the remote servers 400 through 408 for further analysis and review.

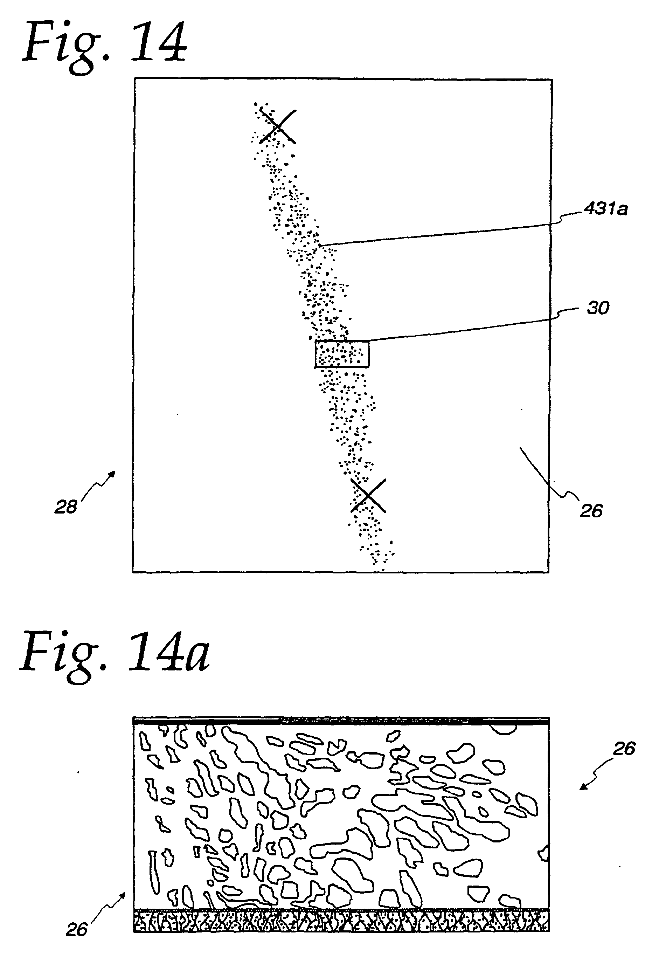

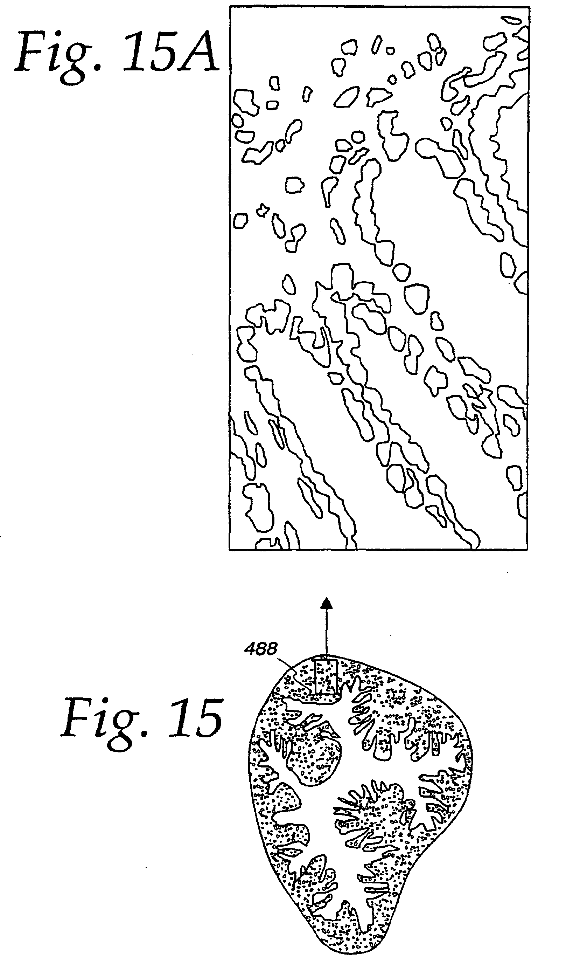

In FIG. 14, there is illustrated on screen 28 a basal layer 431a of a cut cross-section of a rat esophagus. The basal layer is elongated and linear in a downward direction, and the selected point of interest is shown as a box 30 on the basal layer on the composite, low magnification image. The high magnification image 26 of this selected point of interest is shown on screen 26 in FIG. 14A. In FIG. 15 is shown a mouse colon as a reconstructed, low magnification macro image 28 which has been reduced 1/16th in size. The micro image 26 is shown in FIG. 15A, and the marking therefore is shown in FIG. 15.

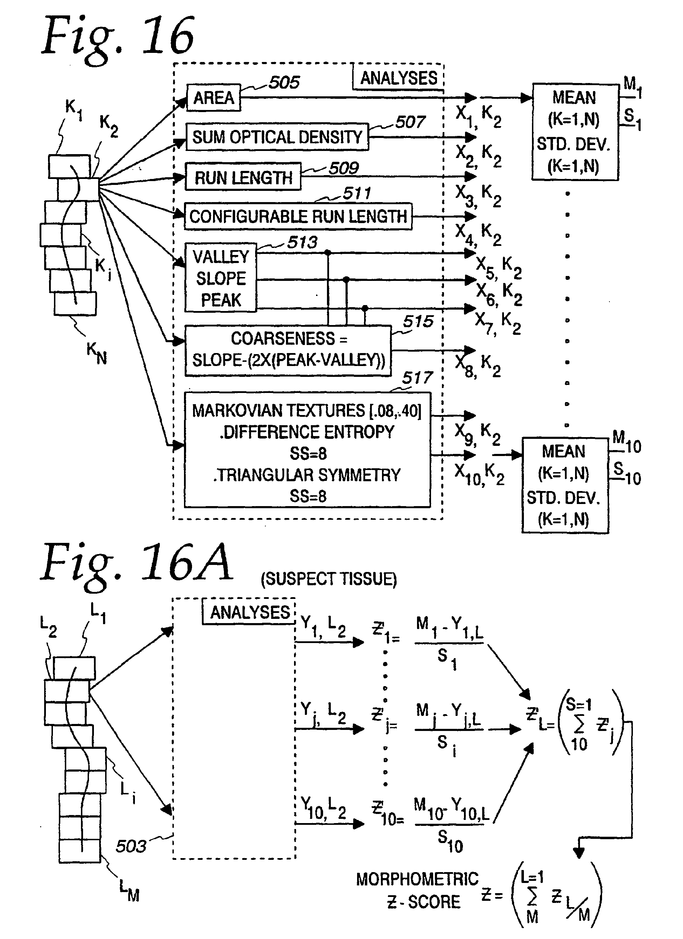

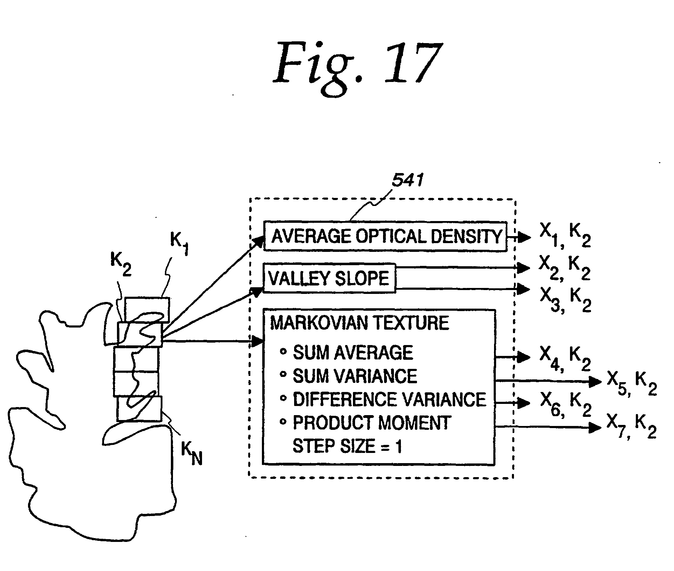

The analysis for texture and for morphological features used to analyze a series of regions 30 on the elongated basal layer that were analyzed at high magnification are shown in FIGS. 16, 16A and 17. The manner of doing these tests and of obtaining a Z score or grade is disclosed in the aforesaid patent application.

While there has been illustrated and described a particular embodiment of the present invention, it will be appreciated that numerous changes and modifications will occur to those skilled in the art.

## Claims

1. A method of using a computer-controlled optical microscope imaging system having a stage movable in the X Y directions with respect to an objective lens to acquire and store multiple resolution images from a specimen (21) on a microscope slide (18), said method comprising the steps of:
placing the specimen (21) and microscope slide (18) on the microscope stage (20) in the computer-controlled microscope imaging system;
digitally scanning by repeatedly, forming a digitized image, and moving the stage (20) to produce a first set of digitized images taken from the specimen (21) on the microscope slide (18)and storing the first set of digitized images, the amount of each stage movement and image size being such as to produce a first set of image tiles which are contiguous, the microscope (16) being at a first optical magnification to allow formation of an overall magnified view (24) of the specimen;
displaying to a user of the computer-controlled microscope imaging system (16) at a remote location the overall view of the specimen;
interactively selecting an area from the overall view at the remote location to be scanned and stored at a second higher optical magnification resolution than the first magnification by drawing around the area to be selected;
computing X and Y co-ordinates of the edges of the selected area and translating the said co-ordinates to stage co-ordinates;
remotely operating the computer-controlled microscope imaging system (16) from the remote location in accordance with the stage coordinates to effect digital scanning and storing of a second set of digitized images (312, 314, 316) of the selected area of the specimen (21) on the microscope slide (18) with the microscope (16) at a second, higher optical magnification than the first magnification and transferring the images to the remote location the second set images comprising contiguous image tiles ; and
addressing the first and second digitized images so that the higher magnification images of the selected areas can be easily located from the lower magnification images.

2. The method of claim 1 further comprising:
marking a selected point on the overall view of the specimen; and
displaying images at the higher magnification resolution for the selected point.

3. The method of claim 1 comprising:
navigating within an elongated specimen by presenting high magnification images for each of a series of selected points along an elongated specimen for display in the overall view.

4. The method of claim 1 further comprising storing a third set of digitized images of the specimen at an intermediate magnification either by a further scan at an intermediate optical magnification, or by reconstruction from the existing high and low magnification digitized images.

5. The method of claim 1 or 4 comprising:
zooming between different magnification views of a selected point of the specimen (21).

6. The method of claim 1 comprising:
presenting the images of the higher magnification image to a display so that the user at the remote location may scroll into view an adjacent contiguous image at the selected higher magnification.

7. The method of claim 1 wherein the images are compressed.

8. The method of claim 1 comprising:
transmitting the images and operating the computer-controlled microscope over the Internet.

9. The method of claim 1 comprising:
storing the digitized images at a server and simultaneously sharing a stored digitized, magnified images with a plurality of viewing devices connected by the Internet.

10. A method in accordance with claim 4 wherein the third set of digitized images is formed, using software, from the data acquired when scanning and storing the second set of higher magnification images.

11. The method of claim 1 further comprising remotely operating the computer-controlled microscope imaging system (16) from the remote location to effect the digital scanning and storing of the first set of digitized images taken from the specimen (21) on the microscope slide (18) in the first set of contiguous image tiles with the microscope (16).

12. A computer-controlled optical microscope imaging system comprising a new line stage (20) for receiving a microscope slide (18) and movable in the X Y directions with respect to an objective lens to acquire and store multiple resolution images from a specimen (21) on the microscope slide (18) ;
means for digitally scanning by repeatedly, forming a digitized image, and moving the stage (20) to produce a first set of digitized images taken from the specimen (21) on the microscope slide (18)and storing the first set of digitized images, the amount of each stage movement and image size being such as to produce a first set of image tiles which are contiguous, the microscope (16) being at a first optical magnification to allow formation of an overall magnified view (24) of the specimen;
means for displaying to a user of the computer-controlled microscope imaging system (16) at a remote location the overall view of the specimen;
means for interactively selecting an area from the overall view at the remote location to be scanned and stored at a second higher optical magnification resolution than the first magnification by drawing around the area to be selected ; new line means for computing X and Y co-ordinates of the edges of the selected area and translating the said co-ordinates to stage co-ordinates; means for remotely operating the computer-controlled microscope imaging system (16) from the remote location in accordance with the stage coordinates to effect digital scanning and storing of a second set of digitized images (312, 314, 316) of the selected area of the specimen (21) on the microscope slide (18) with the microscope (16) at a second, higher optical magnification than the first magnification and transferring the images to the remote location; the second set of images comprising contiguous image tiles ; and
means for addressing the first and second digitized images so that the higher magnification images of the selected areas can be easily located from the lower magnification images.

## Patentansprüche

1. Verfahren der Verwendung eines computergesteuerten optischen Mikroskop-Bildsystems, welches einen bezüglich einer Objektivlinse in X-Y-Richtungen beweglichen Objekttisch aufweist, um Bilder mit mehrfacher Auflösung von einer Probe (21) auf einem Mikroskop-Objektträger (18) aufzunehmen und zu speichern, wobei das Verfahren folgende Schritte aufweist:
Platzieren der Probe (21) und des Mikroskop-Objektträgers (18) auf dem Mikroskop-Objekttisch (20) in dem computergesteuerten optischen Mikroskop-Bildsystem;
digitales Scannen durch wiederholtes Ausbilden digitalisierter Bilder und Bewegen des Objekttisches (20), um einen ersten Satz von der Probe (21) auf dem Mikroskop-Objektträger (18) aufgenommener, digitalisierter Bilder zu erzeugen und Speichern des ersten Satzes digitalisierter Bilder, wobei die Größe jeder Objekttischbewegung und die Bildgröße so ist, dass ein erster Satz zusammenhängender Bildscheiben erzeugt wird und das Mikroskop (16) bei einer ersten optischen Vergrößerung ist, um die Ausbildung einer vergrößerten Gesamtansicht (24) der Probe zu ermöglichen;
Anzeigen der Gesamtansicht der Probe für einen Benutzer des computergesteuerten Mikroskop-Bildsystems (16) an einem entfernten Ort;
interaktives Auswählen eines Gebiets aus der Gesamtansicht von dem entfernten Ort aus, zum Scannen und Speichern mit einer zweiten, höheren optischen Vergrößerungsauflösung als der ersten Vergrößerung, indem um die auszuwählende Fläche herum gezeichnet wird,
Berechnen der X- und Y-Koordinaten der Kanten des gewählten Gebiets und Umrechnen der Koordinaten auf Objekttisch-Koordinaten;
entferntes Bedienen des computergesteuerten Mikroskop-Bildsystems (16) von dem entfernten Ort aus in Überreinstimmung mit den Objekttisch-Koordinaten, um das digitale Scannen und Speichern eines zweiten Satzes digitalisierter Bilder (312, 314, 316) des gewählten Gebietes der Probe (21) auf dem Mikroskop-Objektträger (18) zu bewirken, mit dem Mikroskop (16) bei einer zweiten, höheren optischen Vergrößerung als der ersten Vergrößerung, und Übertragen der Bilder zum entfernten Ort, wobei der zweite Satz von Bildern zusammenhängende Bildscheiben umfasst; und
Kennzeichnen der ersten und zweiten digitalisierten Bilder, so dass die Bilder mit höherer Vergrößerung des gewählten Gebietes leicht von den Bildern mit geringerer Vergrößerung unterschieden werden können.

2. Verfahren nach Anspruch 1, weiterhin umfassend:
Markieren eines gewählten Punktes auf der Gesamtansicht der Probe; und
Darstellen der Bilder bei der höheren Vergrößerungsauflösung für den gewählten Punkt.

3. Verfahren nach Anspruch 1, umfassend:
Navigieren innerhalb einer länglichen Probe durch Zeigen von Bildern mit hoher Vergrößerung für jeden einer Serie von gewählten Punkten entlang der länglichen Probe, zur Darstellung in der Gesamtansicht.

4. Verfahren nach Anspruch 1, welches weiterhin aufweist:
Speichern eines dritten Satzes von digitalisierten Bildern der Probe mit einer Zwischenvergrößerung, entweder durch einen weiteren Scan mit einer optischen Zwischenvergrößerung oder durch die Rekonstruktion aus den bestehenden, digitalisierten Bildern mit hoher und niedriger Vergrößerung.

5. Verfahren nach Anspruch 1 oder 4, umfassend:
Zoomen zwischen verschieden vergrößerten Ansichten eines gewählten Punktes der Probe (21).

6. Verfahren nach Anspruch 1, umfassend:
Darbieten der Bilder des Bildes mit höherer Vergrößerung für einen Bildschirm, und zwar so, dass der Benutzer an dem entfernten Ort ein benachbartes, zusammenhängendes Bild in der gewählten höheren Vergrößerung in die Ansicht scrollen kann.

7. Verfahren nach Anspruch 1, bei dem die Bilder komprimiert sind.

8. Verfahren nach Anspruch 1, welches umfasst:
Übertragen der Bilder und Bedienen des computergesteuerten Mikroskops über das Internet.

9. Verfahren nach Anspruch 1, welches umfasst:
Speichern der digitalisierten Bilder auf einem Server und gleichzeitiges Bereitstellen (engl.: share) der digitalisierten, vergrößerten Bilder für eine Vielzahl von Betrachtungsgeräten, welche durch das Internet verbunden sind.

10. Verfahren nach Anspruch 4, bei dem der dritte Satz digitalisierter Bilder mit Hilfe von Software aus den Daten gebildet wird, welche während des Scannens und Speicherns des zweiten Satzes von Bildern mit höherer Vergrößerung erhalten wurden.

11. Verfahren nach Anspruch 1, welches weiter umfasst:
entferntes Bedienen des computergesteuerten Mikroskop-Bildsystems (16) von einem entfernten Ort aus, um das digitale Scannen und Speichern des ersten Satzes von digitalisierten Bildern, welche von der Probe (21) auf dem Mikroskop-Objektträger (18) im ersten Satz der zusammenhängenden Bildscheiben mit dem Mikroskop (16) aufgenommen wurden, zu bewirken.

12. Computergesteuertes optisches Mikroskop-Bildsystem, welches aufweist:
einen Objekttisch (20) zur Aufnahme eines Mikroskop-Objektträgers (18), welcher bezüglich einer Objektivlinse in X-Y-Richtungen beweglich ist, um Bilder mit mehrfacher Auflösung einer Probe (21) auf dem Mikroskop-Objektträger (18) aufzunehmen und zu speichern;
Mittel zum digitalen Scannen durch wiederholtes Ausbilden digitalisierter Bilder und Bewegungen des Objekttisches (20), um einen ersten Satz von der Probe (21) auf dem Mikroskop-Objektträger (18) aufgenommener, digitalisierter Bilder zu erzeugen und Speichern des ersten Satzes digitalisierter Bilder, wobei die Größe jeder Objekttischbewegung und die Bildgröße so ist, dass ein erster Satz zusammenhängender Bildscheiben erzeugt wird, und das Mikroskop (16) bei einer ersten Vergrößerung ist, um die Ausbildung einer vergrößerten Gesamtansicht (24) der Probe zu ermöglichen;
Mittel zum Anzeigen der Gesamtansicht der Probe für einen Benutzer des computergesteuerten Mikroskop-Bildsystems (16) an einem entfernten Ort;
Mittel zum interaktiven Auswählen eines Gebiets aus der Gesamtansicht von dem entfernten Ort aus, zum Scannen und Speichern mit einer zweiten, höheren optischen Vergrößerungsauflösung als der ersten Vergrößerung, indem um die auszuwählende Fläche herum gezeichnet wird,
Mittel zum Berechnen der X- und Y-Koordinaten der Kanten des gewählten Bereichs und zum Umrechnen der Koordinaten auf Objekttisch-Koordinaten;
Mittel zum entfernten Bedienen des computergesteuerten Mikroskop-Bildsystems (16) von dem entfernten Ort aus in Überreinstimmung mit den Objekttisch-Koordinaten, um das digitale Scannen und Speichern eines zweiten Satzes digitalisierter Bilder (312, 314, 316) des gewählten Gebietes der Probe (21) auf dem Mikroskop-Objektträger (18) zu bewirken, mit dem Mikroskop (16) bei einer zweiten, höheren optischen Vergrößerung als der ersten Vergrößerung, und zum Übertragen der Bilder zum entfernten Ort, wobei der zweite Satz von Bildern aus zusammenhängenden Bildscheiben besteht; und
Mittel zum Kennzeichnen der ersten und zweiten digitalisierten Bilder, so dass die Bilder mit höherer Vergrößerung des gewählten Gebietes leicht von den Bildern mit geringerer Vergrößerung unterschieden werden können.

## Revendications

1. Procédé d'utilisation d'un système d'imagerie de microscope optique commandé par ordinateur possédant un étage pouvant être déplacé dans les directions X Y par rapport à un objectif afin d'acquérir et stocker des images à plusieurs résolutions à partir d'un échantillon (21) sur une lame pour microscope (18), ledit procédé comprenant les étapes consistant à :
placer l'échantillon (21) et la lame pour microscope (18) sur l'étage de microscope (20) dans le système d'imagerie de microscope commandé par ordinateur ;
balayer numériquement en formant de manière répétée une image numérisée, et déplacer l'étage (20) pour produire une première série d'images numérisées prises à partir de l'échantillon (21) sur la lame pour microscope (18) et stocker la première série d'images numérisées, la quantité de chaque déplacement d'étage et la taille de l'image étant sélectionnées de façon à produire une première série de mosaïques d'image qui sont contigües, le microscope (16) étant à un premier grossissement optique pour permettre la formation d'une vue globale grossie (24) de l'échantillon ;
afficher à un utilisateur du système d'imagerie de microscope commandé par ordinateur (16) à une localisation distance la vue globale de l'échantillon ;
sélectionner de manière interactive une zone de la vue globale à la localisation distante à balayer et stocker à une seconde résolution de grossissement optique plus élevée que le premier grossissement en traçant le contour de la zone à sélectionner ;
calculer les coordonnées X et Y des bords de la zone sélectionnée et traduire lesdites coordonnées en coordonnées d'étage ;
utiliser à distance le système d'imagerie de microscope commandé par ordinateur (16) depuis la localisation distante selon les coordonnées d'étage pour effectuer le balayage numérique et stocker une deuxième série d'images numérisées (312, 314, 316) de la zone sélectionnée de l'échantillon (21) sur la lame pour microscope (18) avec le microscope (16) à un second grossissement optique plus élevé que le premier grossissement et transférer les images à la localisation distante, la deuxième série d'images comprenant des mosaïques d'image contigües ; et
adresser les première et seconde images numérisées de façon à ce que les images à grossissement plus important des zones sélectionnées puissent être facilement localisées depuis les images à grossissement plus faibles.

2. Procédé selon la revendication 1 comprenant en outre :
le marquage d'un point sélectionné sur la vue globale de l'échantillon ; et
l'affichage des images à la résolution de grossissement supérieure pour le point sélectionné.

3. Procédé selon la revendication 1 comprenant :
la navigation dans un échantillon allongé par la présentation d'images à fort grossissement pour chacun d'une série de points sélectionnés le long d'un échantillon allongé pour un affichage dans la vue globale.

4. Procédé selon la revendication 1, comprenant en outre le stockage d'une troisième série d'images numérisées de l'échantillon à un grossissement intermédiaire soit par un balayage supplémentaire à un grossissement optique intermédiaire, soit par reconstruction à partir des images numérisées existantes à fort et faible grossissement.

5. Procédé selon la revendication 1 ou 4 comprenant :
un zoom entre différentes vues de grossissement d'un point sélectionné de l'échantillon (21).

6. Procédé selon la revendication 1 comprenant :
la présentation des images de l'image à plus fort grossissement à un dispositif d'affichage de façon à ce que l'utilisation à la localisation distante puisse faire apparaître par défilement une image contigüe adjacente au grossissement supérieur sélectionné.

7. Procédé selon la revendication 1 dans lequel les images sont compressées.

8. Procédé selon la revendication 1 comprenant :
la transmission des images et l'utilisation du microscope commandé par ordinateur sur Internet.

9. Procédé selon la revendication 1 comprenant :
le stockage des images numérisées au niveau d'un serveur et le partage simultané d'une image grossie et numérisée stockée avec une pluralité de dispositifs de visualisation connectés par Internet.

10. Procédé selon la revendication 4 dans lequel la troisième série d'images numérisées est formée, à l'aide d'un logiciel, à partir des données acquises lors du balayage et du stockage de la deuxième série d'images à grossissement supérieur.

11. Procédé selon la revendication 1 comprenant en outre l'utilisation à distance du système d'imagerie de microscope commandé par ordinateur (16) depuis la localisation distante afin d'effectuer le balayage numérique et le stockage de la première série d'images numérisées prises depuis l'échantillon (21) sur la lame pour microscope (18) dans la première série de mosaïques d'image contigües avec le microscope (16).

12. Système d'imagerie de microscope optique commandé par ordinateur comprenant :
un étage (20) pour recevoir une lame pour microscope (18) et pouvant être déplacé dans les directions X Y par rapport à un objectif afin d'acquérir et stocker des images à plusieurs résolutions à partir d'un échantillon (21) sur la lame pour microscope (18) ;
un moyen pour balayer numériquement en formant de manière répétée une image numérisée, et déplacer l'étage (20) pour produire une première série d'images numérisées prises à partir de l'échantillon (21) sur la lame pour microscope (18) et stocker la première série d'images numérisées, la quantité de chaque déplacement d'étage et la taille de l'image étant sélectionnées de façon à produire une première série de mosaïques d'image qui sont contigües, le microscope (16) étant à un premier grossissement optique pour permettre la formation d'une vue globale grossie (24) de l'échantillon ;
un moyen pour afficher à un utilisateur du système d'imagerie de microscope commandé par ordinateur (16) à une localisation distance la vue globale de l'échantillon ;
un moyen pour sélectionner de manière interactive une zone de la vue globale à la localisation distante à balayer et stocker à une seconde résolution de grossissement optique plus élevée que le premier grossissement en traçant le contour de la zone à sélectionner ;
un moyen pour calculer les coordonnées X et Y des bords de la zone sélectionnée et traduire lesdites coordonnées en coordonnées d'étage ;
un moyen pour utiliser à distance le système d'imagerie de microscope commandé par ordinateur (16) depuis la localisation distante selon les coordonnées d'étage pour effectuer le balayage numérique et stocker une deuxième série d'images numérisées (312, 314, 316) de la zone sélectionnée de l'échantillon (21) sur la lame pour microscope (18) avec le microscope (16) à un second grossissement optique plus élevé que le premier grossissement et transférer les images à la localisation distante, la deuxième série d'images comprenant des mosaïques d'image contigües ; et
un moyen pour adresser les première et seconde images numérisées de façon à ce que les images à grossissement plus important des zones sélectionnées puissent être facilement localisées depuis les images à grossissement plus faibles.
